# EUROPÄISCHE PATENTSCHRIFT

(11) **EP 2 044 975 B1**
(45) Veröffentlichungstag und Bekanntmachung des Hinweises auf die Patenterteilung: **22.02.2012**
(21) Anmeldenummer: 07117633.3
(22) Anmeldetag: 01.10.2007
(51) Int. Cl.: A61N 5/10, G06T 7/00

(54) **Verfahren zum Registrieren von 2D-Bilddaten, Computerprogrammprodukt, Navigationsverfahren zum Navigieren eines Behandlungsgerätes im Bereich der Medizin und Recheneinrichtung zum Registrieren von 2D-Bilddaten**
Method for registering 2D image data, computer program product, navigation method for navigating a treatment device in the medical field and computer device for registering 2D image data
Procédé de recalage de données images en 2D, produit de programme informatique, procédé de navigation destiné à la navigation d'un appareil de traitement dans le domaine médical et dispositif de calcul destiné au recalage de données images en 2D

(43) Veröffentlichungstag der Anmeldung: 08.04.2009
(73) Patentinhaber: BrainLAB AG, 85622 Feldkirchen (DE)
(72) Erfinder: Edlauer, Martin, 80809 München (DE); Mezger, Uli, 81543 München (DE); Essenreiter, Robert, 81825 München (DE); Weiser, Manfred, 81825 München (DE)
(74) Vertreter: Schwabe - Sandmair - Marx

(56) Entgegenhaltungen:
- US-A- 4 791 934
- US-A1- 2005 027 187
- PENNEY GRAEME P ET AL: "Validation of a two- to three-dimensional registration algorithm for aligning preoperative CT images and intraoperative fluoroscopy images" MEDICAL PHYSICS, AIP, MELVILLE, NY, US, Bd. 28, Nr. 6, Juni 2001 (2001-06), Seiten 1024-1032, XP012011477 ISSN: 0094-2405
- CLIPPE S ET AL: "Patient setup error measurement using 3D intensity-based image registration techniques" INTERNATIONAL JOURNAL OF RADIATION ONCOLOGY, Bd. 56, Nr. 1, Mai 2003 (2003-05), Seiten 259-265, XP009096386 USA
- ZÖLLEI L: "2D-3D Rigid-Body Registration of X-Ray Fluoroscopy and CT Images" THESIS AT THE MASSACHUSETTS INSTITUTE OF TECHNOLOGY, XX, XX, August 2001 (2001-08), Seiten 1-113, XP007904068
- KALENDER WILLI A.: "Computertomographie. Grundlagen, Gerätetechnologie, Bildqualität, Anwendungen" Juni 2006 (2006-06), PUBLICIS CORPORATE PUBLISHING , XP002470207 * Seite 72 - Seite 75 *
- BEHRENBRUCH C P ET AL: "Fusion of contrast-enhanced breast MR and mammographic imaging data", BRITISH JOURNAL OF RADIOLOGY, vol. 77, 2004, pages S201-S207, ISSN: 0007-1285, DOI: 10.1259/bjr/66587930

## Beschreibung

### Gebiet der Erfindung

Die Erfindung betrifft ein Verfahren zum Registrieren von 2D-Bilddaten, ein Computerprogrammprodukt mit einem Programmcode zum Ausführen des Verfahrens, ein Navigationsverfahren zum Navigieren eines Behandlungsgerätes im Bereich der Medizin, bei dem das erfindungsgemäße Verfahren zum Registrieren von 2D-Bilddaten zugrunde gelegt wird, und eine Recheneinrichtung zum Registrieren von 2D-Bilddaten.

### Stand der Technik

Aus dem Stand der Technik sind bereits unterschiedliche Verfahren zum Registrieren von Bilddaten bekannt. Im Folgenden soll die folgende Definition hinsichtlich Registrierung verwendet werden: Das n-dimensionale Bild eines Körpers ist registriert, wenn jeder räumlichen Position der abgebildeten Punkte ein Bilddatenpunkt zugeordnet ist. Dabei kann es sich um absolute oder relative Positionsangaben handeln; die Wahl des Koordinatensystems ist beliebig bzw. Problem-angepasst. Es können zum Beispiel kartesische, Kugel- oder Zylinderkoordinaten verwendet werden.

Sinn und Zweck von registrierten Bilddaten ist in den allermeisten Fällen die Verwendung der registrierten Bilddatensätze bei navigationsunterstützten Operationen im Bereich der Medizin. Chirurgische Eingriffe, die eine hohe Präzision erfordern, können im Vorfeld präzise geplant und anschließend bildunterstützt durchgeführt werden. Zu diesem Zweck wird im Allgemeinen ein zu navigierendes Behandlungsgerät wie zum Beispiel ein Skalpell mit einer so genannten Markereinrichtung versehen. Diese Markereinrichtung am zu navigierenden Behandlungsgerät wird zugleich mit einer weiteren Markereinrichtung, die sich an einer bekannten Position im Raum befindet, durch ein Kamerasystem erfasst. Die relative Lage zwischen der Markereinrichtung am zu navigierenden Behandlungsgerät einerseits und der zweiten Markereinrichtung andererseits wird ermittelt, und daraus wird auf die Position des zu navigierenden Behandlungsgeräts rückgeschlossen. Auf einer Bildschirmeinheit, die der Operateur während der Operation im Blick hat, wird nun ein registrierter Bilddatensatz dargestellt, in dem gleichzeitig die aktuelle Position des zu navigierenden Behandlungsgeräts dargestellt wird. Diese Art der Darstellung setzt voraus, dass den Bilddatenpunkten ebenfalls räumliche Positionen zugeordnet wurden; Navigation basierend auf einem nicht-registrierten Bilddatensatz ist nicht möglich.

Es gibt unterschiedlichste Bilddatensätze, basierend auf denen navigiert werden kann. So ist es grundsätzlich möglich, auf registrierten 2D-Bilddatensätzen basierend zu navigieren (z.B. basierend auf herkömmlichen Röntgenaufnahmen). Des Weiteren ist es möglich, basierend auf registrierten 3D-Bilddatensätzen zu navigieren, wie sie zum Beispiel bei einer Computertomographie oder bei einer Kernspinresonanzaufnahme (MRT) erzeugt werden. Bei 3D-Bilddatensätzen werden dem Operateur oftmals Schnittdarstellungen durch verschiedene Ebenen auf einem Bildschirm angezeigt.

Nun ist es gelegentlich so, dass verschieden dimensionale Bilddatensätze eines Körpers vorliegen, aber nicht alle Bilddatensätze registriert sind. Konkret ist aus dem Stand der Technik das so genannte "CT-Fluoro-Matching" bekannt (US-Patent 4,791,934 von Brunnett sowie G.P. Penney et al.: "Validation of a two- to three-dimensional registration algorithm for aligning preoperative CT images and intraoperative fluoroscopy images": Medical Physics, Vol. 28, S. 1024-1032, 2001). Dabei liegt ein nicht-registierter 3D-Bilddatensatz wie zum Beispiel eine CT-Aufnahme dem Operateur vor. Im Operationssaal wird ein 2D-Bilddatensatz erzeugt, bei dem die räumliche Lage des Patienten in einem definierten Koordinatensystem bekannt ist, d.h. es wird eine registrierte 2D-Aufnahme wie zum Beispiel bei einem herkömmlichen fluoroskopischen Bild angefertigt. Die Patentschrift lehrt nun, wie die registrierten 2D-Bilddatensätze auf die nicht-registrierten 3D-Datensätze gematcht werden, d.h. es wird eine Übereinstimmung zwischen den Bildern erzeugt. Zu diesem Zweck werden aus den 3D-Bilddaten synthetische 2D-Bilder erzeugt. Dabei kann es sich z.B. um so genannte digitale Rekonstruktionsradiogramme (englisch "digitally reconstructed radiogram", DRR) oder um Schnittbilder (englisch "slice cuts") handeln. Die künstlich erzeugten 2D-Bilder werden nun mit dem registrierten realen 2D-Bilddatensatz verglichen. Der Vergleich erfolgt über eine Ähnlichkeitsmessung, zum Beispiel wird miteinander korrelierte Information jeweils verglichen, andere Möglichkeiten bestehen und sind mittlerweile aus dem Stand der Technik bekannt. Beginnend von einer gegebenen Parametrisierung des Bildsyntheseprozesses wird ein Parametersatz iterativ verändert, bis eine Konfiguration gefunden ist, die die höchste Ähnlichkeit zwischen dem synthetischen 2D-Bild und dem real aufgenommen 2D-Bild, welches registriert ist, liefert. Aus der bekannten räumlichen Position der Bilddatenpunkte im registrierten 2D-Bilddatensatz wird nun rückgeschlossen auf die räumlichen Positionen der Bilddatenpunkte in dem 3D-Bilddatensatz, und eine Registrierung des 3D-Bilddatensatzes ist nunmehr möglich. Die Navigation kann nunmehr basierend auf dem registrierten 3D-Bilddatensatz erfolgen.

Des Weiteren ist aus dem Stand der Technik die DE 103 22 738 A1 bekannt, die ein Verfahren zur markerlosen automatischen Fusion von 2D-Fluoro-C-Bogen-Bildern mit präoperativen 3D-Bildem unter Verwendung eines intraoperativ gewonnen 3D-Datensatzes offenbart. Wie im oben genannten Beispiel des US-Patentes von Brunnett wird auch hier von einem 2D-Bilddatensatz ausgegangen, der registriert ist. Basierend auf den herkömmlich registrierten 2D-Bilddatensätzen wird auf räumliche Positionen von Daten in 3D-Bilddatensätzen zurückgeschlossen, basierend auf denen einerseits eine Operation geplant und andererseits durchgeführt wird. Das genannte Verfahren erfordert allerdings eine zusätzliche 3D-Aufnahme zusätzlich zu einem präoperativ gewonnenen 3D-Bilddatensatz. Der präoperativ gewonnene 3D-Bilddatensatz kann mit einem beliebigen Aufnahmegerät erzeugt werden. Allerdings ist es notwendig, den zusätzlich notwendigen intraoperativen 3D-Bilddatensatz mit demselben Aufnahmegerät zu erzeugen, mit dem auch das registrierte 2D-Bild erzeugt wird. Gemäß der Offenlegungsschrift handelt es sich dabei jeweils um einen C-Bogen, mit dem Röntgenaufnahmen durchgeführt werden. Eine Nutzung der DE 103 227 38 A1 für andere Bildgebungsapparate im Bereich der Medizin ist so nicht möglich.

Es ist eine Aufgabe der Erfindung, ein alternatives Verfahren zum Registrieren von 2D-Bilddaten bereitzustellen, basierend auf denen ein navigationsunterstütztes Operationsverfahren im Bereich der Medizin durchgeführt werden kann.

### Beschreibung der Erfindung

Die Aufgabe wird gelöst durch den Gegenstand der unabhängigen Ansprüche.

Abhängige Ansprüche sind auf besonders vorteilhafte Ausführungsformen der Erfindung gerichtet.

Mit dem erfindungsgemäßen Verfahren zum Registrieren von 2D-Bilddaten ist es möglich, bereits vorhandene nicht-registrierte 2D-Bilddatensätze für Navigationszwecke einzusetzen, nachdem sie dem erfindungsgemäßen Verfahren zum Registrieren unterworfen worden sind. In der Praxis ist es nämlich oftmals so, dass 2D-Bilddatensätze eines Untersuchungsobjektes bereits vorliegen, und es ist dank des erfindungsgemäßen Verfahrens nicht notwendig, erneut eine Aufnahme zum Erhalten eines registrierten 2D-Bilddatensatzes durchzuführen, was die Strahlenbelastung im Falle von zum Beispiel Röntgenaufnahmen für einen Patienten reduziert und Behandlungszeiten insgesamt verkürzt. Erfindungsgemäß ist es vollkommen ausreichend, wenn ein registrierter 3D-Bilddatensatz von dem Untersuchungsobjekt vorliegt.

Vorteilhaft ist es gemäß der Erfindung zum Beispiel möglich, eine herkömmliche fluoroskopische Aufnahme, die mittels eines C-Bogens aufgenommen wurde, nachträglich zu registrieren. Dazu ist lediglich eine registrierte 3D-Aufnahme mittels eines Computertomographen erforderlich. Ist die fluoroskopische 2D-Aufnahme nunmehr registriert, ist es mit Hilfe des erfindungsgemäßen Verfahrens möglich, auch auf Aufnahmeparameter rückzuschließen und das Aufnahmegerät zu charakterisieren.

Außerdem ist es möglich, zweidimensionale Topogramme nachträglich zu registrieren und für Navigationszwecke einzusetzen. Diese zweidimensionalen Topogramme werden zumeist im Vorfeld einer 3D-Komplettaufnahme mittels eines Computertomographen erzeugt, um den Aufnahmebereich festzulegen. Zu diesem Zweck sendet eine Röntgenquelle fächerförmige Detektionsstrahlen aus, d.h. es wird im Wesentlichen eine Ebene und kein dreidimensionaler Raum durchstrahlt, und es wird pro Bild mit dem Fächerstrahl eine Zeile eines 2D-Bilddatensatz generiert. Durch Relativverschiebung des Aufnahmeobjektes zur Röntgenquelle, zum Beispiel durch Hindurchfahren eines Patienten unter der Röntgenquelle, werden mehrere Aufnahmen aus unterschiedlichen Positionen erzeugt, die dann zu einer 2D-Aufnahme zusammengesetzt werden. Die so gewonnen zweidimensionalen Topogramme sind oftmals von sehr guter Bildqualität, und es wäre wünschenswert, auf Basis dieser Aufnahmen eine Navigation durchzuführen.

Ein erfindungsgemäßes Verfahren zum Registrieren von 2D-Bilddaten weist zunächst die Schritte Bereitstellen eines registrierten 3D-Bilddatensatzes eines Untersuchungsobjekts und Bereitstellen eines zu registrierenden 2D-Bilddatensatzes des Untersuchungsobjektes auf. Dabei kann ein bereits vorhandener 3D-Bilddatensatz eines Untersuchungsobjektes bereitgestellt werden; alternativ ist es auch möglich, diesen erst zu erzeugen und anschließend bereitzustellen. Gleiches gilt für den zu registrierenden 2D-Bilddatensatz.

Bei den Bilddatensätzen kann es sich um unterschiedlichste Bilddatensätze handeln. Zum Beispiel kann es sich um Röntgenbilddatensätze oder um Bilddatensätze handeln, die durch Kernspinresonanzaufnahmen erzeugt wurden. Außerdem sind Ultraschallaufnahmen und andere Aufnahmeverfahren denkbar.

Bei dem Untersuchungsobjekt handelt es sich normalerweise um einen Patienten bzw. Teile eines Patienten wie Gliedmaßen, innere Organe oder ein Torso.

In einem weiteren Verfahrensschritt werden synthetische 2D-Bilddatensätze des Untersuchungsobjektes aus dem 3D-Bilddatensatz erzeugt. Anders als in dem bekannten Verfahren des CT-Fluoro-Matching wird erfindungsgemäß eine Parametrisierung der zu erzeugenden synthetischen 2D-Bilddatensätze hinsichtlich Parametern vorgenommen, die den zu registrierenden 2D-Bilddatensatz beschreiben (beim CT-Fluoro-Matching erfolgte eine Parametrisierung bezogen auf die Positionierung des 3D-Bilddatensatzes). Die vorgenommen Parametrisierung dient dazu, unterschiedliche Aufnahmesituationen und eine Konfiguration des Bildgebungsgerätes bei der Erzeugung der synthetischen 2D-Bilddatensätze zu berücksichtigen, die bei der Aufnahme des zu registrierenden 2D-Bilddatensatzes vorgelegen haben könnten. Die genauen Aufnahmeparameter sind bei dem nicht-registrierten 2D-Bilddatensatz zumeist nicht im Vorfeld bekannt.

Zu den Parametern, mittels derer die Parametrisierung erfolgt, zählen zum Beispiel räumliche Parameter wie drei Parameter der Translation und bzw. oder drei Parameter der Rotation. Durch diese Raumtransformationsparameter ist es möglich, die Lage der zu registrierenden 2D-Aufnahme im Raum zu beschreiben. Für ein Röntgenbild etwa beschreiben diese Parameter die Lage und Orientierung der virtuellen Kamera (des Projektionszentrums). Die errechnete Lage bezieht sich dabei auf das Koordinatensystem, in dem die für die Berechnung verwendeten Landmarken oder ähnliches gegeben sind, d.h. zunächst auf ein internes System des 3D-Datensatzes.

Zusätzlich zu den oben beschriebenen Parametern, die die räumliche Lage beschreiben, ist die Parametrisierung durch einen oder mehrere der nachfolgend aufgezählten Parameter vorgenommen: Brennweite, Skalierung, Lage des Bildzentrums. Die Brennweite beschreibt den Abstand der Strahlungsquelle zur Bildebene des zu registrierenden 2D-Bilddatensatzes, wobei ein zentraler Strahl bzw. Hauptachsenstrahl betrachtet wird. Bezüglich dieses Strahles erfolgt die Angabe der Brennweite. Unter der Skalierung des 2D-Bilddatensatzcs wird eine Vergrößerung oder Verkleinerung des Bildes verstanden. Die Skalierung ist bei kartesischen Koordinaten vorteilhaft in beiden Dimensionen des 2D-Bilddatensatzes dieselbe, sie kann aber auch verschieden sein. Ähnliches gilt für 2D-Bilder, denen andere Formen von Koordinatensystemen zugrunde liegen. Die Lage des Bildzentrums beschreibt den Ort im zu registrierenden 2D-Bilddatensatz, auf den der zentrale Abbildungsstrahl bzw. Hauptachsenstrahl abgebildet wird.

Gemäß einer bevorzugten Ausführungsform der Erfindung ist es auch möglich, einen (d.h. mindestens einem) Parameter, der den zu registrierenden 2D-Bilddatensatz beschreibt, einzugeben. Dabei kann es sich um einen der oben aufgeführten Parameter (Raumtransformationsparameter oder zusätzliche Parameter) handeln. Es kann sich auch um einen weiteren Parameter handeln, der es erlaubt, die Aufnahmebedingungen bei der Entstehung des zu registrierenden 2D-Bilddatensatzes in irgendeiner Weise zu charakterisieren.

Erfindungsgemäß werden die parametrisierten synthetischen 2D-Bilddatensätze mit dem zu registrierenden 2D-Bilddatensatz verglichen, und der parametrisierte synthetische 2D-Bilddatensatz mit der größten Ähnlichkeit mit dem zu registrierenden 2D-Bilddatensatz wird aufgefunden. Dabei kommen die an sich bekannten mathematischen Verfahren des Bild-Matching zum Einsatz. Bevorzugt erfolgt das Auffinden und Vergleichen durch iterative Verfahren. Dieses kann zum Beispiel auf einer Optimierungstheorie beruhen.

Ist das parametrisierte synthetische 2D-Bild ermittelt, das die größte Ähnlichkeit mit dem zu registrierenden 2D-Bilddatensatz aufweist, so ist nun auch bekannt, welche Parameter den zu registrierenden 2D-Bilddatensatz beschreiben, d.h. welche Parameter die Aufnahmesituationen bei der Entstehung des ursprünglich nicht registrierten 2D-Bilddatensatzes am besten widerspiegeln bzw. diesen charakterisieren. Gemäß einer bevorzugten Ausführungsform wird wenigstens einer der ermittelten Parameter ausgegeben. Dies kann durch eine Anzeige des Parameters oder der Parameter z.B. auf einem Bildschirm erfolgen. Es ist auch möglich, die ermittelten Parameter mittels einer Druckereinheit auszudrucken.

Das erfindungsgemäße Verfahren zum Registrieren von 2D-Bilddatensätzen ermöglicht es somit, vorher nicht genau charakterisierte bzw. spezifizierte Aufnahmesituation und Aufnahmegeräte zu charakterisieren. Die Kenntnis der Aufnahmeparameter, die im Rahmen des Verfahrens zum Registrieren von 2D-Bilddaten gewonnen worden sind, können bei zukünftigen Aufnahmen mit demselben Aufnahmegerät Berücksichtigung finden. Zum Beispiel ist für zukünftige Aufnahmen nun die Brennweite, die Skalierung oder das Bildzentrum der Aufnahme bekannt.

Nachdem nun der parametrisierte synthetische 2D-Bilddatensatz mit der größten Ähnlichkeit mit dem zu registrierenden 2D-Bilddatensatz aufgefunden worden ist, wird darauf basierend der räumliche Zusammenhang zwischen dem zu registrierenden 2D-Bilddatensatz und dem bereits registrierten 3D-Bilddatensatz ermittelt. Punkte in dem 3D-Bilddatensatz können Punkten in dem 2D-Bilddatensatz zugeordnet werden, und somit ist nun auch der 2D-Bilddatensatz registriert. Da die Positionen von abzubildenden Punkten, die in dem 3D-Bilddatensatz dargestellt worden sind, der registriert ist, bekannt sind, sind auch die Positionen in dem synthetischen parametrisierten 2D-Bilddatensatz bekannt. Diese Kenntnisse lassen sich nun auf den zu registrierenden 2D-Bilddatensatz übertragen.

Vorteilhaft wird eine einzige Abbildung ermittelt, die angewendet auf alle Datenpunkte des zu registrierenden 2D-Bilddatensatzes nunmehr die räumliche Position der abzubildenden Punkte beschreibt. Es ist aber auch möglich, eine Abfolge von Abbildungen anzugeben, die nacheinander angewendet auf alle Datenpunkte des zu registrierenden 2D-Bilddatensatzes nunmehr die räumliche Position der abzubildenden Punkte beschreibt.

Gemäß einer bevorzugten Ausführungsform der Erfindung wird zur Erzeugung der synthetischen 2D-Bilddatensätze ein bilderzeugendes Verfahren simuliert, das einen Kegelstrahl oder einen Fächerstrahl als Detektionsstrahl verwendet. Die geometrischen Verhältnisse, insbesondere Projektionen, die bei einer realen Kegelstrahlaufnahme oder bei einer realen Fächerstrahlaufnahme auftreten, werden dabei simuliert. Es erfolgen bei der Simulation dabei zum Beispiel Summationen von Werten von Datenpunkten entlang der jeweiligen Einzelstrahlrichtung, d.h. in der jeweiligen Projektionsrichtung. Ein Kegelstrahl im Sinne der Erfindung ist dabei durch einen räumlichen Öffnungswinkel charakterisiert, und der Kegelstrahl durchstrahlt einen dreidimensionalen Raumabschnitt. Im Gegensatz dazu ist ein Fächerstrahl gemäß der Erfindung durch einen ebenen Auffächerungswinkel charakterisiert, und der Strahl durchstrahlt im Wesentlichen eine Fläche. Während der Kegelstrahl pro Aufnahme mittels eines Kegelstrahles bereits ein zweidimensionales Bild ergibt, wird ein 2D-Bilddatensatz, der mittels Fächerstrahlen aufgenommen wird, aus mehreren Einzelaufnahmen des Fächerstrahles erst zusammengesetzt. Ein Fächerstrahl ist somit ein Strahl, der in einer Richtung eine starke Aufweitung aufweist, und der in der dazu orthogonalen Richtung stark fokussiert ist bzw. nur eine äußerst geringe vernachlässigbare Aufweitung aufweist.

Gemäß einer bevorzugten Ausführungsform der Erfindung handelt es sich bei dem zu registrierenden 2D-Bilddatensatz um ein fluoroskopische Aufnahme, die mittels eines C-Bogens aufgenommen wurde, wobei zur Erzeugung der synthetischen 2D-Bilddatensätze ein Kegelstrahl als Detektionsstrahl simuliert wird.

Gemäß einer anderen bevorzugten Ausführungsform der Erfindung handelt es sich bei dem zu registrierenden 2D-Bilddatensatz um ein zweidimensionales Topogramm, das mit einem CT-Scanner aufgenommen wurde, wobei zur Erzeugung der synthetischen 2D-Bilddatensätze ein Fächerstrahl als Detektionsstrahl simuliert wird. Hinsichtlich der zitierten zweidimensionalen Topogramme wird auf die obigen Ausführungen zu diesem Aufnahmetyp verwiesen.

Gemäß einem weiteren Aspekt der Erfindung bezieht sich diese auf ein Computerprogrammprodukt mit einem Programmcode zum Ausführen des beschriebenen erfindungsgemäßen Verfahrens zum Registrieren von 2D-Bilddaten. Bei diesem Computerprogrammprodukt kann es sich zum Beispiel um eine CD-ROM oder eine DVD handeln; auch ist es möglich, den Programmcode auf einem Memorystick abzuspeichern. Andere Speichermedien sind denkbar. Der Programmcode kann in einer beliebigen Programmiersprache verfasst sein. Zum Beispiel ist es möglich, einen Programmcode in C oder C++ oder aber in einer Skriptsprache abzufassen.

Gemäß einem weiteren Aspekt der Erfindung betrifft diese ein Navigationsverfahren zum Navigieren eines Behandlungsgerätes im Bereich der Medizin. Wie oben bereits beschrieben, sind Navigationsverfahren an sich bekannt. Das beanspruchte Navigationsverfahren weist nun die folgenden Schritte auf: Registrieren eines 2D-Bilddatensatzes gemäß dem oben beschriebenen Verfahren zum Registrieren von 2D-Bilddaten; bildliches Darstellen einer Position eines zu navigierenden Behandlungsgerätes in dem registrierten 2D-Bilddatensatz; und Positionieren des zu navigierenden Behandlungsgerätes unter Zuhilfenahme der bildlichen Darstellung. Den Kern des beanspruchten Navigationsverfahrens bildet also der erfindungsgemäß gewonnene registrierte 2D-Bilddatensatz. Dieser registrierte 2D-Bilddatensatz wird während einer Operation bildlich dargestellt, und die Position des zu navigierenden Behandlungsgerätes wie zum Beispiel eines Skalpells wird ebenfalls in dem registrierten 2D-Bilddatensatz dargestellt. Dazu kann der gesamte Bilddatensatz oder aber Ausschnitte davon dargestellt werden. Die gleichzeitige Darstellung von navigiertem Behandlungsgerät und registriertem 2D-Bilddatensatz unterstützt nun den Operateur beim Positionieren des zu navigierenden Behandlungsgerätes.

Gemäß einem weiteren Aspekt der Erfindung bezieht sich diese auf eine Recheneinrichtung zum Registrieren von 2D-Bilddaten. Bei dieser Recheneinrichtung kann es sich zum einen um einen Computer handeln. Zum anderen ist es auch möglich, dass es sich um eine in eine andere Vorrichtung integrierte CPU handelt. Die Recheneinrichtung weist eine Inputeinheit und eine Bearbeitungseinheit auf. Die Inputeinheit ist eingerichtet, um einen registrierten 3D-Bilddatensatz eines Untersuchungsobjekts bereitzustellen und um einen zu registrierenden 2D-Bilddatensatz des Untersuchungsobjektes bereitzustellen. Dabei kann die Einheit auf vorab abgespeicherte 3D-Bilddatensätze und 2D-Bilddatensätze zurückgreifen. Es ist aber auch möglich, dass diese Datensätze in die Inputeinheit erst eingegeben werden, zum Beispiel an diese übermittelt werden, z.B. in abgespeicherter Form auf einem Datenträger. Auch eine Übermittlung über Netzwerke ist denkbar.

Die Bearbeitungseinheit ist eingerichtet, um synthetische 2D-Bilddatensätze des Untersuchungsobjektes aus dem 3D-Bilddatensatz zu erzeugen, wobei eine Parametrisierung der zu erzeugenden synthetischen 2D-Bilddatensätze hinsichtlich Parametern erfolgt, die den zu registrierenden 2D-Bilddatensatz beschreiben. Des Weiteren ist die Bearbeitungseinheit eingerichtet, um die parametrisierten synthetischen 2D-Bilddatensätze mit dem zu registrierenden 2D-Bilddatensatz zu vergleichen und um den parametrisierten synthetischen 2D-Bilddatensatz mit der größten Ähnlichkeit mit dem zu registrierenden 2D-Bilddatensatz aufzufinden. Schließlich ist die Bearbeitungseinheit eingerichtet, um darauf basierend den räumlichen Zusammenhang zwischen dem zu registrierenden 2D-Bilddatensatz und dem registrierten 3D-Bilddatensatz zu ermitteln und somit den 2D-Bilddatensatz zu registrieren. Hinsichtlich der Einzelheiten betreffend die Bearbeitungseinheit und hinsichtlich der bevorzugten Ausführungsformen betreffend die Bearbeitungseinheit wird auf die Beschreibung des erfindungsgemäßen Verfahrens zum Registrieren von 2D-Bilddaten verwiesen. Das dabei Ausgesagte gilt in uneingeschränktem Umfang auch für die erfindungsgemäße Recheneinrichtung bzw. für die in diesem Paragraphen beschriebene Bearbeitungseinheit.

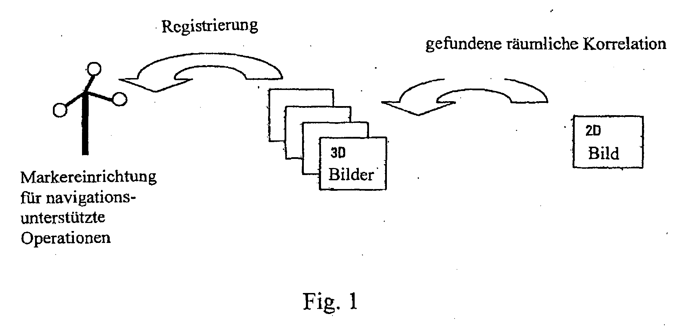

Die vorliegende Erfindung wird bei Betrachtung der beigefügten Figur 1 noch besser verstanden werden. Dabei zeigt:
- Fig. 1:: Matching bzw. Zuordnen eines 2D-Bilddatensatzes zu einem 3D-Bilddatensatz, um die Verwendung des 2D-Bilddatensatzes für navigationsunterstützte Operationen zu er- möglichen.

In der Mitte von Fig. 1 ist ein 3D-Bilddatensatz dargestellt. Dieser 3D-Bilddatensatz setzt sich im Regelfall aus mehreren Schnittaufnahmen zusammen, was durch die hintereinander angeordneten Einzelbilder dargestellt werden soll.

Der 3D-Bilddatensatz ist ein registrierter 3D-Bilddatensatz. Bei der Aufnahme des 3D-Bilddatensatzes wurde jeweils eine Markereinrichtung, die links im Bild dargestellt ist, mit abgebildet. Die Position der Markereinrichtung bzw. der einzelnen Punkte, die die Marker darstellen, im Raum ist dabei bekannt. Bei den einzelnen Markern der Markereinrichtung kann es sich um aktive oder passive Marker handeln, die entweder selbst Strahlung eines bestimmten Typs aussenden, die mit einem geeigneten Empfänger aufgefangen wird, oder aber die Marker können Strahlung reflektieren, die von einer (nicht dargestellten) Strahlenquelle ausgesendet wird, die reflektierte Strahlung wird wiederum mit einem geeigneten Detektor detektiert. Aufgrund der detektierten Strahlung wird die Position der Markereinrichtung ermittelt. Üblicherweise handelt es sich zum Beispiel um passive Marker, die Infrarotstrahlung reflektieren.

Da die Position der Markereinrichtung bei jeder einzelnen Schnittaufnahme, die zu dem 3D-Bilddatensatz beiträgt, bekannt ist, ist für jede der Schnittaufnahmen bekannt, wie die Punkte im Raum, an denen sich die Marker befinden, in den jeweiligen Bestandteilen des 3D-Bilddatensatzes dargestellt werden. Auf diese Weise wird also ein registrierter 3D-Bilddatensatz erhalten.

Mit dem erfindungsgemäßen Verfahren zum Registrieren von 2D-Bilddaten wird nun eine räumliche Korrelation zwischen dem 3D-Bilddatensatz und dem 2D-Bilddatensatz aufgefunden. Die räumliche Korrelation kann zum Beispiel durch eine oder mehrere Abbildungsfunktionen beschrieben werden. Ist nun aber die räumliche Korrelation zwischen dem 3D-Bilddatensatz und dem 2D-Bilddatensatz bekannt, so kann daraus auf die räumliche Position der in dem 2D-Bilddatensatz abgebildeten Punkte rückgeschlossen werden. Es wird auf diese Weise also ein registriertes 2D-Bild erhalten, bzw. bei dem 2D-Bilddatensatz handelt es sich nach Anwendung des erfindungsgemäßen Verfahrens um einen registrierten 2D-Bilddatensatz.

## Patentansprüche

1. Verfahren zum Registrieren von zu registrierenden 2D-Bilddaten, das die folgenden Schritte aufweist:
• Bereitstellen eines registrierten 3D-Bilddatensatzes eines Untersuchungsobjekts;
• Bereitstellen des zu registrierenden 2D-Bilddatensatzes des Untersuchungsobjektes;
• Erzeugen von synthetischen 2D-Bilddatensätzen des Untersuchungsobjektes aus dem 3D-Bilddatensatz, wobei eine Parametrisierung der zu erzeugenden synthetischen 2D-Bilddatensätze hinsichtlich Parametern erfolgt, die den zu registrierenden 2D-Bilddatensatz beschreiben und Aufnahmeparameter sind, die dazu dienen, unterschiedliche Aufnahmesituationen und eine Konfiguration des Bildgebungsgerätes zu berücksichtigen, die bei der Aufnahme des zu registrierenden 2D-Bilddatensatzes vorgelegen haben könnten;
• Vergleichen der parametrisierten synthetischen 2D-Bilddatensätze mit dem zu registrierenden 2D-Bilddatensatz und Auffinden des parametrisierten synthetischen 2D-Bilddatensatzes mit der größten Ähnlichkeit mit dem zu registrierenden 2D-Bilddatensatz;
• darauf basierend Ermitteln des räumlichen Zusammenhangs zwischen dem zu registrierenden 2D-Bilddatensatz und dem registrierten 3D-Bilddatensatz und somit Registrierung des 2D-Bilddatensatzes,
wobei
die Aufnahmeparameter durch das Auffinden gewonnen werden und wenigstens einer der Aufnahmeparameter, der dem parametrisierten synthetischen 2D-Bilddatensatz mit der größten Ähnlichkeit zu dem zu registrierenden 2D-Bilddatensatz zuzuordnen ist, ausgegeben wird und wobei die durch das Auffinden gewonnenen Aufnahmeparameter einen oder mehrere der folgenden Parameter umfassen: Brennweite, Skalierung, Lage des Bildzentrums, wobei folgende Definition hinsichtlich Registrierung verwendet wird: Das n-dimensionale Bild eines Körpers ist registriert, wenn jeder räumlichen Position der abgebildeten Punkte ein Bilddatenpunkt zugeordnet ist.

2. Verfahren gemäß Anspruch 1, wobei das Auffinden und Vergleichen durch ein iteratives Verfahren erfolgt.

3. Verfahren gemäß einem der vorangehenden Ansprüche, wobei die Parametrisierung durch zusätzlich drei Parameter der Translation und/oder drei Parameter der Rotation erfolgt.

4. Verfahren gemäß einem der vorangehenden Ansprüche, das zusätzlich den folgenden Schritt aufweist:
• Eingeben wenigstens eines Parameters, der den zu registrierenden 2D-Bilddatensatz beschreibt.

5. Verfahren gemäß einem der vorangehenden Ansprüche, wobei zur Erzeugung der synthetischen 2D-Bilddatensätze ein bilderzeugendes Verfahren, das einen Kegelstrahl oder einen Fächerstrahl als Detektionsstrahl verwendet, simuliert wird.

6. Verfahren gemäß Anspruch 5, wobei es sich bei dem zu registrierenden 2D-Bilddatensatz um eine fluoroskopische Aufnahme handelt, die mittels eines C-Bogens aufgenommen wurde, und wobei zur Erzeugung der synthetischen 2D-Bilddatensätze ein Kegelstrahl als Detektionsstrahl simuliert wird.

7. Verfahren gemäß Anspruch 5, wobei es sich bei dem zu registrierenden 2D-Bilddatensatz um ein zweidimensionales Topogramm handelt, das mit einem CT-Scanner aufgenommen wurde, und wobei zur Erzeugung der synthetischen 2D-Bilddatensätze ein Fächerstrahl als Detektionsstrahl simuliert wird.

8. Computerprogrammprodukt mit einem Programmcode zum Ausführen des Verfahrens gemäß einem der Ansprüche 1 bis 7.

9. Navigationsverfahren zum Navigieren eines Behandlungsgerätes im Bereich der Medizin, das die folgenden Schritte aufweist:
• Registrieren eine 2D-Bilddatensatzes gemäß dem Verfahren gemäß einem der Ansprüche 1 bis 7;
• Bildliches Darstellen einer Position eines zu navigierenden Behandlungsgerätes in dem registrierten 2D-Bilddatensatz;
• Positionieren des zu navigierenden Behandlungsgeräts unter Zuhilfenahme der bildlichen Darstellung.

10. Recheneinrichtung zum Registrieren von zu registrierenden 2D-Bilddaten
• mit einer Inputeinheit, die eingerichtet ist,
o um einen registrierten 3D-Bilddatensatz eines Untersuchungsobjekts bereitzustellen; und
o um den zu registrierenden 2D-Bilddatensatz des Untersuchungsobjektes bereitzustellen;
• und mit einer Bearbeitungseinheit, die eingerichtet ist,
o um synthetische 2D-Bilddatensätze des Untersuchungsobjektes aus dem 3D-Bilddatensatz zu erzeugen, wobei eine Parametrisierung der zu erzeugenden synthetischen 2D-Bilddatensätze hinsichtlich Parametern erfolgt, die den zu registrierenden 2D-Bilddatensatz beschreiben und Aufnahmeparameter sind, die dazu dienen, unterschiedliche Aufnahmesituationen und eine Konfiguration des Bildgebungsgerätes zu berücksichtigen, die bei der Aufnahme des zu registrierenden 2D-Bilddatensatzes vorgelegen haben könnten;
o um die parametrisierten synthetischen 2D-Bilddatensätze mit dem zu registrierenden 2D-Bilddatensatz zu vergleichen und um den parametrisierten synthetischen 2D-Bilddatensatz mit der größten Ähnlichkeit mit dem zu registrierenden 2D-Bilddatensatz aufzufinden; und
o um darauf basierend den räumlichen Zusammenhang zwischen dem zu registrierenden 2D-Bilddatensatz und dem registrierten 3D-Bilddatensatz zu ermitteln und somit den 2D-Bilddatensatz zu registrieren,
wobei
die Aufnahmeparameter durch das Auffinden gewonnen werden und wenigstens einer der Aufnahmeparameter, der dem parametrisierten synthetischen 2D-Bilddatensatz mit der größten Ähnlichkeit zu dem zu registrierenden 2D-Bilddatensatz zuzuordnen ist, ausgegeben wird
und wobei die durch das Auffinden gewonnenen Aufnahmeparameter einen oder mehrere der folgenden Parameter umfassen: Brennweite, Skalierung, Lage des Bildzentrums,
wobei folgende Definition hinsichtlich Registrierung verwendet wird: Das n-dimensionale Bild eines Körpers ist registriert, wenn jeder räumlichen Position der abgebildeten Punkte ein Bilddatenpunkt zugeordnet ist.

## Claims

1. A method for registering two-dimensional image data to be registered, the method comprising the steps of:
• providing a registered three-dimensional image data set of an object under examination;
• providing the two-dimensional image data set of the object under examination, which is to be registered;
• generating synthetic two-dimensional image data sets of the object under examination from the three-dimensional image data set, wherein the synthetic two-dimensional image data sets to be generated are parameterised with regard to parameters which describe the two-dimensional image data set to be registered and are recording parameters which serve to take into account different recording situations and a configuration of the imaging apparatus which could have applied when the two-dimensional image data set to be registered was recorded;
• comparing the parameterised synthetic two-dimensional image data sets with the two-dimensional image data set to be registered, and finding the parameterised synthetic two-dimensional image data set having the greatest similarity to the two-dimensional image data set to be registered;
• on the basis of this, ascertaining the spatial relationship between the two-dimensional image data set to be registered and the registered three-dimensional image data set, and thus registering the two-dimensional image data set,
wherein the recording parameters are acquired by the finding step and at least one of the parameters to be assigned to the parameterised synthetic two-dimensional image data set having the greatest similarity to the two-dimensional image data set to be registered is outputted,
wherein the recording parameters acquired by the finding step include one or more of the following parameters: focal length, scaling, position of the centre of the image, and wherein the following definition with regard to registration is used: the n-dimensional image of a body is registered when an image data point has been assigned to every spatial position of the mapped points.

2. The method in accordance with claim 1, wherein the finding and comparing steps are performed using an iterative method.

3. The method in accordance with any one of the preceding claims, wherein parameterisation is performed by additionally using three parameters of translation and/or three parameters of rotation.

4. The method in accordance with any one of the preceding claims, additionally comprising the step of:
• inputting at least one parameter which describes the two-dimensional image data set to be registered.

5. The method in accordance with any one of the preceding claims, wherein an image-generating method which uses a conical or fanned ray as a detection ray is simulated in order to generate the synthetic two-dimensional image data sets.

6. The method in accordance with claim 5, wherein the two-dimensional image data set to be registered is a fluoroscopic recording which has been recorded by means of a C-arc, and wherein a conical ray is simulated as a detection ray in order to generate the synthetic two-dimensional image data sets.

7. The method in accordance with claim 5, wherein the two-dimensional image data set to be registered is a two-dimensional topogram which has been recorded by a CT scanner, and wherein a fanned ray is simulated as a detection ray in order to generate the synthetic two-dimensional image data sets.

8. A computer program product comprising a program code for performing the method in accordance with any one of claims 1 to 7.

9. A navigation method for navigating a treatment apparatus in the medical field, comprising the steps of:
• registering a two-dimensional image data set in accordance with the method in accordance with any one of claims 1 to 7;
• visually displaying a position of a treatment apparatus to be navigated, in the registered two-dimensional image data set;
• positioning the treatment apparatus to be navigated with the aid of the visual display.

10. A computational device for registering two-dimensional image data to be registered, comprising:
• an input unit configured to:
o provide a registered three-dimensional image data set of an object under examination; and
o provide the two-dimensional image data set of the object under examination, which is to be registered;
• and a processing unit configured to:
o generate synthetic two-dimensional image data sets of the object under examination from the three-dimensional image data set, wherein the synthetic two-dimensional image data sets to be generated are parameterised with regard to parameters which describe the two-dimensional image data set to be registered and are recording parameters which serve to take into account different recording situations and a configuration of the imaging apparatus which could have applied when the two-dimensional image data set to be registered was recorded;
o compare the parameterised synthetic two-dimensional image data sets with the two-dimensional image data set to be registered and find the parameterised synthetic two-dimensional image data set having the greatest similarity to the two-dimensional image data set to be registered; and
o on the basis of this, ascertain the spatial relationship between the two-dimensional image data set to be registered and the registered three-dimensional image data set, and thus register the two-dimensional image data set,
wherein the recording parameters are acquired by the finding step and at least one of the parameters to be assigned to the parameterised synthetic two-dimensional image data set having the greatest similarity to the two-dimensional image data set to be registered is outputted,
wherein the recording parameters acquired by the finding step include one or more of the following parameters: focal length, scaling, position of the centre of the image, and wherein the following definition with regard to registration is used: the n-dimensional image of a body is registered when an image data point has been assigned to every spatial position of the mapped points.

## Revendications

1. Procédé pour enregistrer des données d'image 2D qui doivent être enregistrées, comportant les étapes suivantes consistant à :
• fournir un ensemble de données d'image 3D enregistré d'un objet en cours d'examen,
• fournir l'ensemble de données d'image 2D à enregistrer de l'objet en cours d'examen,
• générer des ensembles de données d'image 2D synthétiques de l'objet en cours d'examen à partir de l'ensemble de données d'image 3D, dans lequel les ensembles de données d'image 2D synthétiques à générer sont paramétrés en ce qui concerne des paramètres qui décrivent l'ensemble de données d'image 2D à enregistrer et qui sont des paramètres d'enregistrement qui servent à prendre en compte différentes situations d'enregistrement et une configuration de l'appareil d'imagerie, qui ont pu exister lors de l'enregistrement de l'ensemble de données d'image 2D à enregistrer,
• comparer les ensembles de données d'image 2D synthétiques paramétrés à l'ensemble de données d'image 2D à enregistrer et trouver l'ensemble de données d'image 2D synthétique paramétré qui présente la plus grande similitude avec l'ensemble de données d'image 2D à enregistrer,
• sur la base de ceci, déterminer la relation spatiale entre l'ensemble de données d'image 2D à enregistrer et l'ensemble de données d'image 3D enregistré et enregistrer ainsi l'ensemble de données d'image 2D,
dans lequel les paramètres d'enregistrement ont été acquis lors de la recherche et au moins un des paramètres d'enregistrement, qui doit être associé à l'ensemble de données d'image 2D synthétique paramétré présentant la plus grande similitude avec l'ensemble de données d'image 2D à enregistrer, est généré,
et dans lequel les paramètres d'enregistrement acquis lors la recherche incluent un ou plusieurs des paramètres suivants : distance focale, mise à l'échelle, position du centre d'image,
dans lequel la définition suivante concernant l'enregistrement est utilisée : l'image de dimension n d'un corps est enregistrée lorsqu'un point de donnée d'image est associé à chaque position spatiale des points reproduits en image.

2. Procédé selon la revendication 1, dans lequel la recherche et la comparaison sont effectuées par un procédé itératif.

3. Procédé selon l'une quelconque des revendications précédentes, dans lequel le paramétrage s'effectue au moyen de trois paramètres de translation supplémentaires et/ou de trois paramètres de rotation supplémentaires.

4. Procédé selon l'une quelconque des revendications précédentes, comportant de plus l'étape suivante consistant à :
• entrer au moins un paramètre qui décrit l'ensemble de données d'image 2D à enregistrer.

5. Procédé selon l'une quelconque des revendications précédentes, dans lequel un procédé de génération d'image est simulé, lequel procédé utilise un rayon conique ou un rayon en éventail comme rayon de détection, afin de générer les ensembles de données d'image 2D synthétiques.

6. Procédé selon la revendication 5, dans lequel l'ensemble de données d'image 2D à enregistrer est un enregistrement fluoroscopique qui a été enregistré au moyen d'un arceau en C, et dans lequel un rayon conique est simulé en tant que rayon de détection afin de générer les ensembles de données d'image 2D synthétiques.

7. Procédé selon la revendication 5, dans lequel l'ensemble de données d'image 2D à enregistrer est un topogramme bidimensionnel qui a été enregistré avec un tomodensitomètre, et dans lequel un rayon en éventail est simulé en tant que rayon de détection afin de générer les ensembles de données d'image 2D synthétiques.

8. Produit de programme informatique comportant un code de programme pour mettre en oeuvre le procédé selon l'une quelconque des revendications 1 à 7.

9. Procédé de navigation pour déplacer un appareil de traitement dans le domaine de la médecine, comportant les étapes suivantes consistant à :
• enregistrer un ensemble de données d'image 2D selon le procédé selon l'une quelconque des revendications 1 à 7,
• représenter en image une position d'un appareil de traitement à déplacer dans l'ensemble de données d'image 2D enregistré,
• positionner l'appareil de traitement à déplacer à l'aide de la représentation en image.

10. Dispositif de calcul pour enregistrer des données d'image 2D à enregistrer, comportant :
• une unité d'entrée configurée pour :
- fournir un ensemble de données d'image 3D enregistré d'un objet en cours d'examen, et
- fournir l'ensemble de données d'image 2D à enregistrer de l'objet en cours d'examen,
• et une unité de traitement configurée pour :
- générer des ensembles de données d'image 2D synthétiques de l'objet en cours d'examen à partir de l'ensemble de données d'image 3D, dans lequel les ensembles de données d'image 2D synthétiques à générer sont paramétrés en ce qui concerne des paramètres qui décrivent l'ensemble de données d'image 2D à enregistrer et qui sont des paramètres d'enregistrement qui servent à prendre en compte différentes situations d'enregistrement et une configuration de l'appareil d'imagerie, qui ont pu exister lors de l'enregistrement de l'ensemble de données d'image 2D à enregistrer,
- comparer les ensembles de données d'image 2D synthétiques paramétrés à l'ensemble de données d'image 2D à enregistrer et rechercher l'ensemble de données d'image 2D synthétique paramétré qui présente la plus grande similitude avec l'ensemble de données d'image 2D à enregistrer,
- sur la base de ceci, déterminer la relation spatiale entre l'ensemble de données d'image 2D à enregistrer et l'ensemble de données d'image 3D enregistré et enregistrer ainsi l'ensemble de données d'image 2D,
dans lequel les paramètres d'enregistrement ont été acquis lors de la recherche et au moins un des paramètres d'enregistrement, qui doit être associé à l'ensemble de données d'image 2D synthétique paramétré présentant la plus grande similitude avec l'ensemble de données d'image 2D à enregistrer, est généré,
et dans lequel les paramètres d'enregistrement acquis lors la recherche incluent un ou plusieurs des paramètres suivants : distance focale, mise à l'échelle, position du centre d'image,
dans lequel la définition suivante concernant l'enregistrement est utilisée : l'image de dimension n d'un corps est enregistrée lorsqu'un point de donnée d'image est associé à chaque position spatiale des points reproduits en image.
